# EUROPEAN PATENT APPLICATION

(11) **EP 2 090 887 A2**
(43) Date of publication of application: **19.08.2009**
(21) Application number: 09152452.0
(22) Date of filing: 10.02.2009
(51) Int. Cl.: G01N 33/18, G01N 27/07

(54) **Hand-held systems and methods for detection of contaminants in a liquid**

(30) Priority: 15.02.2008 US 32196
(71) Applicant: GE-Hitachi Nuclear Energy Americas LLC, Wilmington, NC 28401 (US)
(72) Inventor: Potyrailo, Radislav Alexandrovich, Niskayuna, NY 12309 (US); Spears, Brian Forrest, Hampstead, NC 28443 (US); Monk, David James, Rexford, NY 12148 (US)
(74) Representative: Gray, Thomas

(57) **Abstract**

A method for detecting contaminants in a liquid is provided. The method can include filling at least a portion of a sample container interior chamber with a liquid sample and submerging a sensor probe of a hand-held portable sensing device in a liquid sample. The method can additionally include sensing an electrical conductivity of the liquid sample utilizing at least one conductivity sensor and automatically selecting a particular one of a plurality of contaminant concentration detection (CCD) algorithms based on the sensed conductivity. The method can further include setting a sensitivity of at least one ionic species sensor to a sensitivity level particular to the selected CCD algorithm and sensing non-desired contaminants in the liquid sample utilizing the at least one ionic species sensor. A concentration of the non-desired contaminant in the liquid sample is then determined in accordance with the selected CCD algorithm.

## Description

### FIELD

The present teachings relate to systems and methods for detecting and quantifying contaminants in a liquid.

### BACKGROUND

The statements in this section merely provide background information related to the present disclosure and may not constitute prior art.

The detection of trace (e.g., less than 1% by volume) and microtrace (e.g., less than 1.0 x 10⁻⁴ % by volume) levels of chemical contaminants in aqueous solutions is important for monitoring the condition of numerous applications. For example, ultrapure water (i.e. water having a microtrace concentration of ionic species) is desirable in many industrial processes including, but not limited to, the semiconductor, pharmaceutical, agricultural, chemical, energy, and food processing industries. In one specific example, nuclear reactors can employ ultrapure water for cooling purposes. The ultrapure water can comprise contaminants, which cause corrosion and other problems in the reactor's coolant and moderator systems.

The detection of chemical contaminants has evolved significantly over the last few decades. There are several techniques currently available for the detection and quantification of trace levels of ionic species in aqueous solutions. These techniques include ion chromatography (IC), inductively coupled plasma atomic emission spectrometry (ICP), mass spectrometry (MS), Inductively Coupled Plasma Mass Spectrometry (ICP-MS), and capillary electrophoresis (CE). Additionally, electrochemical, optical, and hybrid chemical sensors (e.g., combinations of different techniques such as surface plasmon resonance with anodic stripping voltammetry), have been applied for trace analysis of ionic species in water. Unfortunately, these methods can require extensive sample preparation or are limited by poor selectivity, inadequate detection limits, interference effects, baseline drift, and contamination during sampling or handling.

### SUMMARY

In accordance with one aspect of the present disclosure, a method for detecting contaminants in a liquid is provided. In various embodiments, the method can include filling at least a portion of a sample container interior chamber with a liquid sample and submerging a sensor probe of a hand-held portable sensing device in a liquid sample. The method can additionally include sensing an electrical conductivity of the liquid sample utilizing at least one conductivity sensor and automatically selecting a particular one of a plurality of contaminant concentration detection (CCD) algorithms based on the sensed conductivity. The method can further include setting a sensitivity of at least one ionic species sensor to a sensitivity level particular to the selected CCD algorithm and sensing non-desired contaminants in the liquid sample utilizing the at least one ionic species sensor. A concentration of the non-desired contaminant in the liquid sample is then determined in accordance with the selected CCD algorithm.

In accordance with another aspect of the present disclosure, a hand-held, portable system for detection of contaminants in a liquid is provided. In various embodiments, the system can include a sample container for retaining a liquid sample, and a hand-held portable sensing device that is at least partially submersible into the liquid sample and operable for determining a concentration of a non-desired contaminant in the liquid sample. In various implementations the hand-held portable sensing can include at least one conductivity sensor for sensing an electrical conductivity of the liquid sample, at least one ionic species sensor for sensing the non-desired contaminant in the liquid sample, and a controller electrically connected to the at least one conductivity sensor and the at least one ionic species sensor. In various embodiments, the controller can include a processor, and a computer readable memory device having stored thereon a contaminant concentration detection (CCD) algorithm selection routine. The CCD algorithm selection routine is executable by the processor to determine an electrical conductivity value of the liquid sample and, based on the determined conductivity value, instruct the processor to execute a particular one of a plurality of CCD algorithms stored on the memory device. Each respective CCD algorithm is configured to determine a concentration of the non-desired contaminant in the liquid sample using a respective different sensitivity setting for the at least one ionic species sensor.

Further areas of applicability of the present teachings will become apparent from the description provided herein. It should be understood that the description and specific examples are intended for purposes of illustration only and are not intended to limit the scope of the present teachings.

### DRAWINGS

There follows a detailed description of embodiments of the invention by way of example only with reference to the accompanying drawings in which:

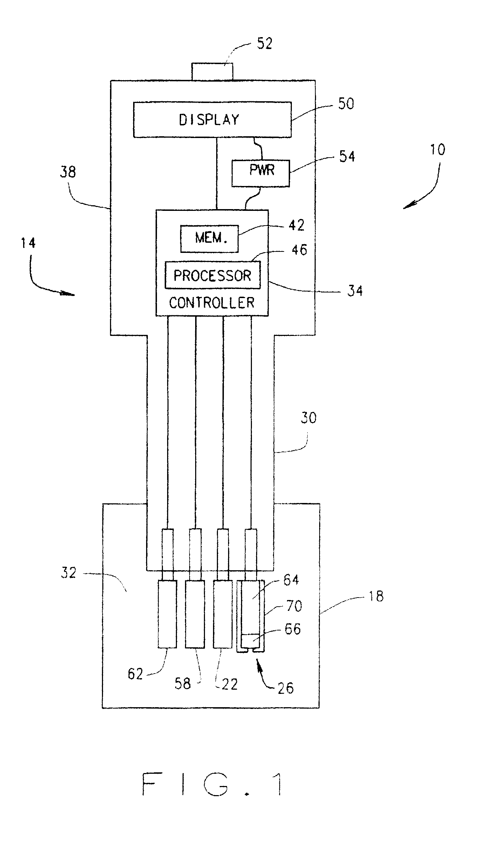

Figure 1 is a block diagram of a hand-held, portable contaminant detection system for determining a concentration of one or more non-desired contaminants in a liquid sample, in accordance with various embodiments of the present disclosure;

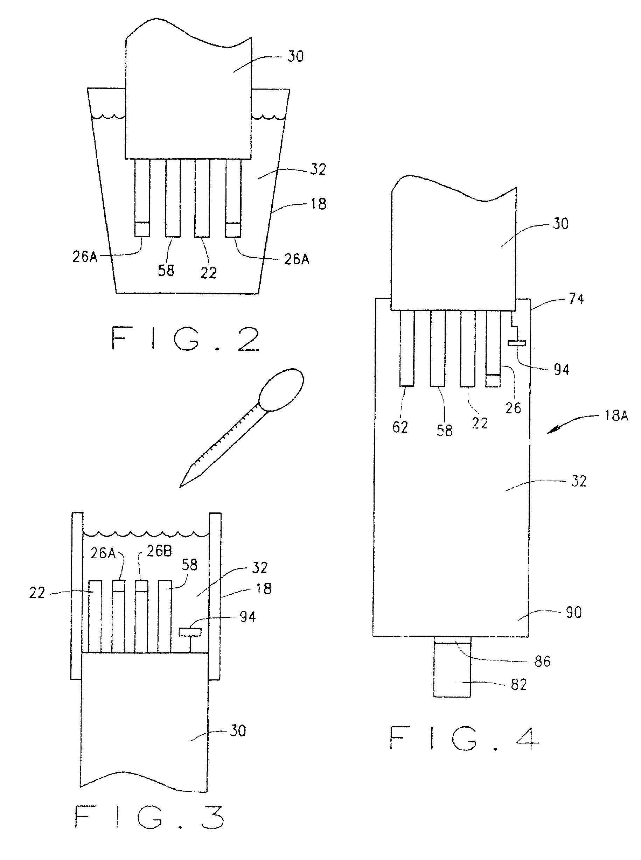

Figure 2 is a sectional view of a hand-held, portable sensor device (HHPSD) of the hand-held, portable contaminant detection system, shown in Figure 1, illustrating an ionic sensor array submerged in a liquid sample retained in liquid sample container, in accordance with various embodiments of the present disclosure;
Figure 3 is a sectional view of a hand-held, portable sensor device (HHPSD) of the hand-held, portable contaminant detection system, shown in Figure 1, illustrating an ionic sensor array submerged in a liquid sample retained in liquid sample container, in accordance with various other embodiments of the present disclosure; and
Figure 4 is a sectional view of a hand-held, portable sensor device (HHPSD) of the hand-held, portable contaminant detection system, shown in Figure 1, illustrating an ionic sensor array submerged in a liquid sample retained in liquid sample container, in accordance with yet other various embodiments of the present disclosure.

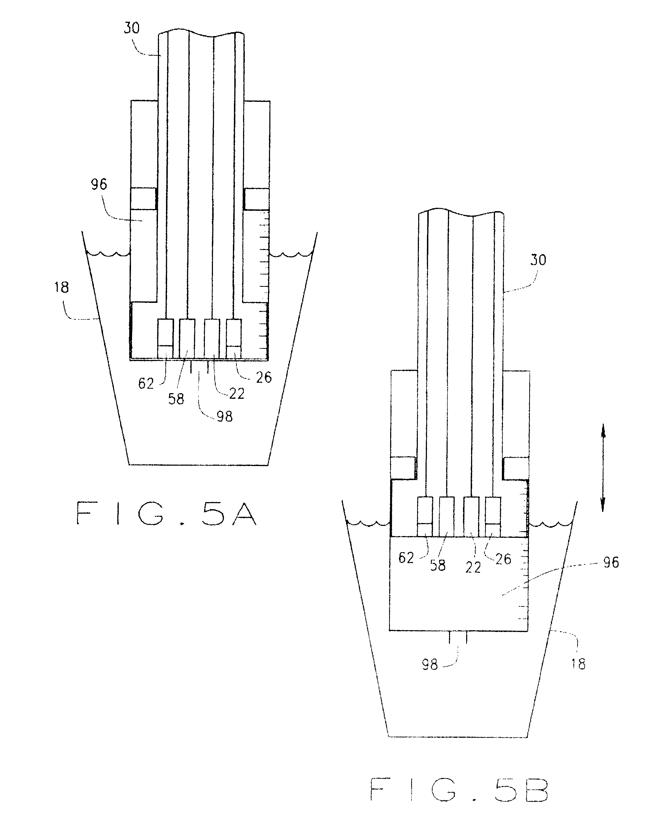

Figures 5A and 5B are sectional views of a hand-held, portable sensor device (HHPSD) of the hand-held, portable contaminant detection system shown in Figure 1, illustrating an ionic sensor array in accordance with yet other various embodiments of the present disclosure.

### DETAILED DESCRIPTION

The following description is merely exemplary in nature and is in no way intended to limit the present teachings, application, or uses. Throughout this specification, like reference numerals will be used to refer to like elements.

Figure 1 is an exemplary illustration of a hand-held, portable contaminant detection system 10 for detection of one or more particular non-desired contaminants in a very pure, i.e., very low concentration of contaminants, liquid sample and calculation of a concentration of the non-desired contaminant(s) within the liquid sample. In accordance with various embodiments, the contaminant detection system 10 includes a hand-held, portable sensing device (HHPSD) 14 operable to determine a concentration of a non-desired contaminant in the very pure liquid sample, and a hand-held, portable liquid sample container 18 for retaining the liquid sample. In various implementations, the HHPSD 14 includes one or more conductivity sensors 22 and one or more ionic species sensors 26 attached to, or extending from, a sensor probe 30 of the HHPSD 14. During use and operation of the HHPSD 14, as described below, at least a portion of the sensor probe 30, e.g., a distal end having the conductivity sensor(s) 22 and the ionic species sensor(s) 26 attached to or extending therefrom, is submerged in the liquid sample retained within an interior chamber 32 of the sample container 18.

Although the HHPSD 14 can include one or more of each of the conductivity sensors 22 and ionic species sensors 26, for clarity and simplicity, unless otherwise disclosed, the HHPSD 14 will be described herein as including a single conductivity sensor 22 and a single ionic species sensor 26.

Each of the conductivity sensor 22 and the ionic sensor 26 are electrically connected to a controller 34 housed within a head 38 of the HHPSD 14, from which the sensor probe 30 extends. The controller 34 is operable to control all the operations, functions and features of the HHPSD 14 described herein. For example, once the sensor probe 30 is submerged in the liquid sample, the ionic sensor 26 senses one or more non-desired contaminants, e.g., ionic species, in the liquid sample and provides the sensed readings to the controller 34, which utilizes the readings to determine the concentration of the non-desired contaminant(s) in the liquid sample. Generally, the controller 34 includes a computer readable electronic memory device 42 having stored thereon various programs, data, code, information and algorithms that are accessed, executed, utilized and/or implemented by a processor 46 of the controller 34. Alternatively, the electronic memory device 42 can be a separate component electrically connected to controller 34. Or, in other embodiments, the electronic memory device can comprise a connection port, e.g., a USB port, fire-wire port or memory stick slot, electrically connected to the controller 34, for electrically connecting a removable electronic memory device 42, e.g., a thumb drive or memory stick, to the controller 34. In various embodiments, the HHPSD 14 can additionally include a display 50, e.g., a liquid crystal display (LCD), for displaying various numbers, values, readings, ranges, etc., sensed, calculated and/or generated by HHPSD 14, as described herein. In alternate embodiments, the controller can be connected to an external, supplemental processor for additional analytic capabilities by means of a connection port, e.g. USB port, fire-wire port, BNC connector, by means of radio frequency waves, or by means of optical or infrared signal.

In various embodiments, the HHPSD 14 further includes a stored power device 54 for providing electrical energy, e.g., DC current and voltage, to the controller 34, processor 46, memory device 42, display 50, conductivity sensor 22, ionic species sensor 26 and all other sensors, devices and components of the HHPSD 14 described herein. The stored power device 54 can be any stored power device suitable to provide the energy requisite for operation of the HHPSD 14, as described herein. For example, in various embodiments, the stored power device 54 can be one or more replaceable batteries, rechargeable batteries, or capacitors.

In various implementations, the HHPSD 14 can additionally include a temperature sensor 58 electrically connected to the controller 34 for sensing a temperature of the liquid sample and/or an organic carbon sensor 62 electrically connected to the controller for sensing an amount of organic carbon in the liquid sample, e.g., a total amount of organic carbon in the sample.

Generally, the ionic sensor 26 comprises a transducer 64 that is disposed in contact with a film 66. A surface of the film 66 that is opposite the surface in contact with the transducer 64 is disposed in fluid communication with the liquid sample when the sensor probe 30 is submerged in the liquid sample, which comprises trace levels, e.g., small quantities of contaminants (not shown). The transducer 64 is electrically connected to the controller 34 to provide electrical communication there between. As described above, during use, the ionic sensor 26 provides information to the controller 34 that is utilized to determine the concentration of one or more non-desirable contaminants within the liquid sample.

More particularly, the film 26 is constructed or fabricated to allow the passage of only one or more non-desired contaminants, e.g., the ions of the contaminant(s) that are to be evaluated by the HHPSD 14, and restrict or prevent the passage of all other contaminants through the film 66, e.g., the additional contaminants in the liquid sample that are not to be evaluated. The transducer 64 senses the non-desired ionic species that pass through the film 66 and provides electrical information, or readings, to the controller 34, indicating an amount or quantity of the non-desired ions that pass through the film 66. Then, based on the volume of the sample liquid being tested, the controller determines a concentration of the non-desired ionic species, i.e., non-desired contaminant(s), in the liquid sample.

In various embodiments, the volume of the liquid sample retained within the interior chamber 32 of the container 18 and needed for use with the HHPSD 14 is less than 10,000 microliters. For example, in various implementations, the volume of the liquid sample retained within the container 18 and needed for use with the HHPSD 14 is between approximately 0.001 and 10,000 microliters. In other exemplary embodiments, the volume of the liquid sample retained within interior chamber 32 of the container 18 and needed for use with the HHPSD 14 can be between approximately 0.01 and 5,000 microliters. While, in yet other exemplary embodiments, the volume of the liquid sample retained within the container 18 and needed for use with the HHPSD 14 can be between approximately 0.1 and 1,000 microliters.

In various embodiments, the film 66 can comprise a polymeric material that is chosen based on its ability to allow the passage of the specific contaminant there through. To be more specific, an exemplary polymeric material employed for the film 66 can have a glass transition temperature that is below the temperature at which the sensor will operate, thereby providing a semi-viscous state, which enables the diffusion of the specific contaminants there through. Additives can be incorporated within the polymeric materials employed for the film 66 to tailor the diffusion of ionic species in the film 66. The polymer matrix can also be doped with an ion exchange material having a positive or negative charge. Therefore, the ionic sensor 26 can be selected to include a film 66 having a specific ion exchange material based on the charge of the contaminants within the liquid sample that are to be prevented from passing through the film 66. For example, ion exchange materials having a negative charge can be used to prevent positively charged contaminants from passing through the film 66, and positively charged ion exchange materials can be used to prevent negatively charged contaminants from passing through the film 66. Additionally, a neutral charged film 66 can be used for contaminants having a neutral charge.

Furthermore, in various embodiments, the composition of the film 66 can be fabricated to provide a selective binding process of an ion of interest using ionophores. Ionophores are added to the polymeric material to increase the selectivity of the film 66 and to further facilitate the transport of the non-desirable contaminant to be sensed through the film. Still further, the thickness of the film 66 can affect the ionic transport there through. Thus, for each different ionic species desired to be sensed by the HHPSD 14 a different ionic species sensor 26 must be utilized having a film 66 constructed or fabricated to allow only passage of the non-desirable contaminant(s) to be sensed.

Therefore, in various embodiments, the ionic species sensor 26 is removably connected to the HHPSD probe 30. Thus, a single HHPSD 14 can be employed to test for many different non-desired contaminants by merely installing a particular ionic species sensor 26 constructed to sense a particular one of various different non-desired contaminants. In various other embodiments, the ionic sensor 26 and other sensors, e.g., the conductivity sensor 22, the temperature sensor 58 and the organic carbon sensor 62, can be components of a removable sensor module that can be removably connected from the HHPSD probe 30 by a threaded connection, friction fitting, etc., to enable replacement of the sensors and reuse of the HHPSD probe 30.

Alternatively, in various embodiments, the ionic species sensor 26 can be fixedly mounted to the HHPSD 14 such that a different HHPSD 14 must be employed to test for each different one or more non-desired contaminants. The transducer 64 can be any electrochemical transducer suitable to provide information to the controller 34 that can be utilized to determine the concentration of a contaminant within the liquid sample. For example, the transducer 64 can be any transducer that operates based on the principle that the electrical properties measured by the controller 34 increases with the quantity of ions that pass through the film 66 and contact the transducer 64. The electrical properties measured can be complex impedance at multiple frequencies, electrochemically-modulated impedance, electrical current, and electrical potential, or any combination thereof. Nonlimiting examples of a suitable transducer include electrochemical transducers are potentiometric, amperometric, impedometric, field-effect transistors, and others.

In various embodiments, the ionic sensor 26 can include a manifold 70 having the transducer 64 and film 66 disposed within the manifold 70. The manifold 70 is employed to secure the transducer 64 and film 66. However, it is to be apparent that the manifold 70 is not necessary in applications wherein the film 66 is bonded to the transducer 64.

Operation of the hand-held, portable contaminant detection system 10 will now be described. To determine the concentration of one or more particular non-desirable contaminants within a large volume of liquid, e.g., the ultra-pure water used in a boiling water nuclear reactor (BWR), a sample of the liquid is drawn from the large volume and retained within interior chamber 32 of the liquid sample container 18.

The distal end HHPSD sensor probe 30 having the conductivity sensor 22 and the ionic sensor 26 attached thereto, or extending therefrom, is then submerged into the liquid sample. Once the sensor probe 30 and the conductivity and ionic sensors 22 and 26 have been submerged in the liquid sample, the controller 34 can be enabled, or activated, i.e., the HHPSD 14 is turned 'ON', to begin analysis of the liquid sample and calculation of the concentration of the non-desired contaminant(s) therein.

In accordance with various embodiments, the computer readable electronic memory device 42 has stored thereon a contaminant concentration detection (CCD) algorithm selection routine and a plurality of CCD algorithms. The CCD algorithm selection routine is configured to determine an electrical conductivity value of the liquid sample utilizing conductivity readings from the conductivity sensor 22, and based on the determined conductivity value, instruct the processor to execute a particular one of the plurality of CCD algorithms. Each of the respective CCD algorithms is configured to determine a concentration of a non-desired contaminant in the liquid sample using a respective different sensitivity setting for ionic species sensor 26.

As used herein, the phase "configured to determine" when used in reference to algorithms and routines stored on the electronic memory device 42 (e.g., the CCD algorithms and CCD algorithm selection routine), should be understood to mean that execution of the respective algorithm or routine by the processor 46 will result in the controller 34, providing, calculating or generating the described "configured to" action. For example, the phrase "each of the respective CCD algorithms is configured to determine a concentration of a non-desired contaminant in the liquid sample using a respective different sensitivity setting for ionic species sensor 26" will be understood to mean that execution of each of the respective CCD algorithms by the processor 46 will result in the controller 34 determining a concentration of a non-desired contaminant in the liquid sample using a respective different sensitivity setting for ionic species sensor 26.

Once the conductivity sensor 22 and ionic species sensor have been submerged in the liquid sample and the controller 34 has been enabled, the controller implements the CCD algorithm selection routine. That is, the processor 46 executes the CCD algorithm selection routine. During implementation of the CCD algorithm selection routine, the controller 34 acquires one or more conductivity readings from the conductivity sensor 22 indicating the conductivity of the liquid sample. The conductivity of the liquid sample is generally based on the concentration of impurities in the liquid sample. The higher the impurity concentration, the higher the conductivity. However, in very pure liquids, such as the very pure water used in a BWR the conductivity will typically be very low because the concentration of impurities is very low. Furthermore, since the concentration of impurities in the very pure sample liquid will be very small, slight variations in the concentration level can have a large impact on whatever the liquid is being used for. For example, very slight variances in the concentration levels of impurities in the water used in a BWR can have a significant negative impact on the, longevity and maintenance expense of the BWR. Accordingly, to sense and determine accurately the concentration of one or more non-desired contaminants, the sensitivity, or resolution, of the ionic species sensor 26 must be appropriately set to sense the very low, trace, levels of the particular non-desired contaminant(s).

As set forth above, each CCD algorithm is configured, i.e., structured or written, to determine a concentration of a non-desired contaminant in the liquid sample using a respective different sensitivity setting for ionic species sensor 26. More particularly, each CCD algorithm is configured to set, or adjust, the sensitivity of the ionic species sensor 26 to a respective particular level. That is, execution of each respective CCD algorithm will set, or adjust, the sensitivity of the ionic species sensor 26 to a particular level specific to the respective CCD algorithm being implemented, i.e., executed. For example, a first CCD algorithm can be configured to set the ionic species sensor 26 to sense an impurity or contaminant concentration of 0 ppb to 50 ppb (parts per billion), while a second CCD algorithm can be configured to set the ionic species sensor 26 to sense an impurity or contaminant concentration of 50 ppb to 500 ppb, while yet a third CCD algorithm can be configured to set the ionic species sensor 26 to sense an impurity or contaminant concentration of 500 ppb to 5,000 ppb, and so on.

Thus, once the conductivity sensor 22 and ionic species sensor have been submerged in the liquid sample, the controller implements the CCD algorithm selection routine to determine the conductivity level of the liquid sample. Subsequently, based on the determined conductivity of the liquid sample, the CCD selection routine automatically selects an appropriate CCD algorithm that will set the sensitivity of the ionic species sensor 26 to correspond with the determined conductivity of the liquid sample. For example, if the conductivity of the liquid sample is determined to be approximately 0, the CCD selection routine can automatically direct the controller 34 to execute a CCD algorithm that will set the sensitivity level of the ionic species sensor 26 to test for contaminates having a concentration of 0 ppb to 50 ppb. However, for example, if the conductivity of the liquid sample is determined to be approximately 200 ppb, the CCD selection routine can automatically direct the controller 34 to execute a CCD algorithm that will set the sensitivity level of the ionic species sensor 26 to test for contaminates having a concentration of 50 ppb to 500 ppb.

Thus, execution of the CCD algorithm selection routine will sense and determine the conductivity level of the liquid sample. Then, based on the determined conductivity, the CCD algorithm selection routine will automatically instruct the controller 34 to implement a particular one of the CCD algorithms. Where after, the selected CCD algorithm will set the ionic species sensor 26 to a corresponding appropriate sensitivity level and determine the concentration of the non-desired contaminant(s) utilizing that sensitivity setting. As described above, the particular non-desired contaminant(s) that is/are sensed and the concentration thereof calculated, is/are determined by the selection of the particular selected ionic species sensor 26 having the appropriate film 66 fabricated to allow passage there through of only the particular non-desired contaminant(s) of interest, i.e., the particular contaminant(s) to be sensed.

Generally, once the selected CCD algorithm adjusts the sensitivity of the ionic species sensor 26 to the respective setting, execution of the selected CCD algorithm utilizes readings from the ionic species sensor 26 to calculate the concentration of the particular non-desired contaminant(s). In various embodiments, each of the CCD algorithms is configured to utilize temperature readings from the temperature sensor 58, in addition to the readings from the ionic species sensor 26, to determine temperature-based affects on the concentration of the particular non-desirable contaminant(s). Additionally, in various other embodiments, each of the CCD algorithms are configured to utilize organic carbon readings from the organic carbon sensor 62, in addition to the readings from the ionic species sensor 26 and the temperature sensor 58, to determine the concentration of the particular non-desirable contaminant(s).

Referring now to Figures 2 and 3, as described above, the liquid sample is drawn from the larger volume of the liquid, e.g., water from a nuclear reactor core, and retained within the interior chamber 32 of the liquid sample container 18. More particularly, to accurately determine the concentration of a particular contaminant within the liquid sample, the volume of the drawn liquid sample must be known. Thus, a particular volume of the liquid, e.g., 1.0 milliliter or 100 microliters, is drawn to provide the liquid sample. In various embodiments, the drawn liquid sample can be deposited and retained within the interior chamber 32 of a beaker type liquid sample container 18, such as that exemplarily illustrated in Figure 2. As used herein, the term 'beaker' includes any container with a desired volume, e.g., a micro-well, flask, nano-well, etc. The ionic species sensor 26, the conductivity sensor 22, and all other sensors described herein, e.g., the temperature sensor 58 and/or the organic carbon sensor 62, are then submerged in the liquid sample by placing the HHPSD probe 30 into the liquid sample.

Alternatively, as exemplarily illustrated in Figure 3, in various other embodiments, the sample container 18 can be connected to the HHPSD probe 30 to encompass the ionic species sensor 26, the conductivity sensor 22, and all other sensors described herein, within the container 18. The liquid sample can then be drawn from the larger volume, using a suitable device such as a pipette, and deposited into the interior chamber 32 of the sample container 18 to submerge the sensors, e.g., the ionic species sensor 26, the conductivity sensor 22, etc.

Referring now to Figure 4, in various implementations, it can be desirable to prevent exposure of the drawn sample to ambient impurities, e.g., air-borne gases (e.g., CO₂) and particulate matter, to maintain the integrity of the liquid sample. Accordingly, in various embodiments, the sample container 18 can comprise an air-tight container 18A attachable at a first end 74 to the HHPSD probe 30 in an air-tight fashion having the conductivity sensor 22, the ionic species sensor 26 and any other applicable sensors described herein, positioned within the interior chamber 32 of the sample container 18A. In various embodiments, the HHPSD 14 can be structured to include the air-tight container 18A as a single unit having the probe 30 pre-connected to the first end 74 in an air-tight fashion. Alternatively, in other embodiments, the HHPSD 14 and the air-tight container 18A can be separate components wherein the probe 30 is coupled, e.g., threaded, friction fitted, etc., to the first end 74 in an air-tight fashion.

The sample container 18A additionally includes a tubular stem 82 and air-tight seal 86 at an opposing second end 90. Furthermore, the sample container 18A is manufactured such that interior chamber 32 is under a vacuum. To draw the liquid sample and submerge the ionic species sensor 26, the conductivity sensor 22, and all other sensors described herein, e.g., the temperature sensor 58 and/or the organic carbon sensor 62, the tubular stem 82 is exposed to the larger volume of the liquid and the seal 86 is broken. Accordingly, when the seal 86 is broken the vacuum within the interior chamber 32 will draw liquid sample into the interior chamber 32 thereby, preventing exposure of the liquid sample to ambient impurities and preserving the integrity of the liquid sample. The air-tight container 18A, more particularly the interior chamber 32, is sized and the vacuum created such that a particular volume of the liquid is drawing into the interior chamber 32.

Referring now to Figure 5A and 5B, in various implementations, to prevent exposure of the drawn sample to ambient impurities, e.g. air-borne gases (e.g., CO2) and particulate matter, to maintain the integrity of the liquid sample, the sample container 18 can comprise a cylinder 96, with the HHPSD probe 30 having the conductivity sensor 22, the ionic species sensor 26 and any other applicable sensors described herein positioned within the cylinder 96 in an air-tight fashion. The cylinder 96 can then be filled by withdrawing the probe 30 which serves an additional function as a piston. Withdrawing the piston/probe 30 will create a vacuum within the cylinder 96 and draw the sample through a small opening or valve 98 into the proximity of the sensors at a specified volume either set by graduations on the side of the cylinder 96, hard stops within the cylinder 96, or by electronic measurements of piston/probe 30 stroke. The cylinder 96 can be cleared by pushing the piston/probe 30 to the base of the cylinder 96.

Referring now to Figure 3, 4, 5A and 5B, in various instances, it may be desirable to take contamination reading, i.e., ionic species readings, via the ionic species sensor 26 over a period of time. For example, if the conductivity reading is very low, it may be necessary and desirable to take several ionic species readings over a specific period of time. Thus, the CCD algorithms can be configured to acquire several ionic species readings over the specific period of time and calculate the contaminant concentration utilizing the multiple ionic species readings.

Additionally, in such instances, it may be desirable, or more particularly, necessary for accurate contaminant concentration calculations, to maintain or stabilize the liquid sample at a substantially constant temperature during the specific period over which the multiple readings will be acquired. Accordingly, in various embodiments, the HHPSD 14 can further include a thermal electric heating and cooling device 94 electrically connected to the controller 34 for heating and cooling the liquid sample. Therefore, in instances where maintaining the liquid sample at a substantially constant temperature is desired and/or necessary, each of the CCD algorithms are configured to control the operation of the thermal electric heating and cooling device 94 and temperature sensor 58 to maintain the liquid sample at a substantially constant temperature over the specific period of time. The respective CCD algorithms can then acquire multiple ionic species readings over the period to accurately calculate the contaminant concentration utilizing the multiple ionic species readings.

In still other instances, it may be desirable and/or necessary to cycle the temperature of the liquid sample between two or more temperatures over a particular period of time to generate an accurate contaminant concentration calculation. Therefore, in various embodiments, each of the CCD algorithms are configured to control the operation of the thermal electric heating and cooling device 94 and temperature sensor 58 to cycle the temperature of the liquid sample between two or more temperatures over the specific period of time. The respective CCD algorithms can then acquire multiple ionic species readings at each of the temperatures over the period to accurately calculate the contaminant concentration utilizing the multiple ionic species readings.

Referring now to Figures 1, 2, 3, 4, 5A and 5B, as described above, in various implementations, the HHPSD 14 can include a display, e.g., a liquid crystal display (LCD), for displaying various numbers, values, readings, ranges, etc., sensed, calculated and/or generated by HHPSD 14. For example, each of the CCD algorithms can be configured to display, via the display 50, any one or more of the sensed conductivity of the liquid sample, the temperature of the liquid sample, the amount of organic carbon in the liquid sample, the time elapsed or remaining for a specific sensing period or increment thereof, and the final contaminant concentration calculation. As additionally described above, in various embodiments, the HHPSD 14 can include a stored power device 54 operable to provide electrical energy, e.g., DC current and voltage, to any one or more of the controller 34, processor 46, memory device 42, display 50, conductivity sensor 22, ionic species sensor 26, the temperature sensor 58, the organic carbon sensor 62, thermal electric heading and cooling device 94 and any other sensors, devices and components of the HHPSD 14.

In order to conserve the energy usage of the stored power device 54, in various embodiments, each of the CCD algorithms can include a power saving subroutine for controlling power consumption by the controller 34, and any one or more of the conductivity sensor 22, the ionic species sensor 26, the temperature sensor 58, the organic carbon sensor 62, the thermal electric heating and cooling device 94 and the display 50 during use of the hand-held portable sensing device. For example, the CCD algorithms can be configured to display readings and values for a limited time, turn off the thermal electric heating and cooling device 94 when it is not needed to heat or cool the liquid sample, turn off the temperature sensor 58 when it is not needed, turn off the organic carbon sensor when it is not needed, etc.

Referring now to Figures 2 and 3, in various embodiments, the HHPSD 14 can include a plurality of ionic species sensors 26 that form an array of ionic species sensors 26 attached to the sensor probe 30. For simplicity, Figures 2 and 3 exemplarily illustrate only a first ionic species sensor 26A and second ionic species sensor 26B that form an ionic species sensor array. However, it should understood that in various embodiments, the HHPSD 14 can include more than two ionic species sensors 26, i.e., 26A, 26B, 26C, etc., that form the array. In various implementations each ionic species sensor 26 independently senses respective independent values of the non-desired contaminant in the liquid sample, and communicates the independent values to the controller 34 for calculation by the CCD algorithms of the contaminant concentration within the liquid sample.

For example, the sensor array 50 can be employed to determine an average contaminant concentration within the liquid sample. That is, each ionic species sensor 26 of the array can independently provide electrical information to the controller 34, whereby the respective CCD algorithm utilizes the ionic species reading from each of the ionic species sensors 26A, 26B, etc., to calculate an average contaminant concentration. In such embodiments, the ionic species sensors 26 can be configured similar to one another, e.g., having the same film 66.

In alternative embodiments, the sensor array can be capable of supplying electrical information to the controller 34 based upon the time dependent migration of the non-desired contaminant through the film 66. To be more specific, the duration of time required for the non-desired ion to pass through a film 66 can be affected by presence and/or concentration of other contaminants within the liquid sample. Therefore, the ionic species sensors 26A, 26B, etc., can comprise films 66 of the same material(s) that differ in thicknesses. In such configurations, the duration of time required for the electrical information supplied by each ionic species sensor 26 to reach a plateau, or reach a specific level, can be evaluated and utilized by the respective CCD algorithm to determine if other contaminants are affecting the ion transport of the non-desired contaminant of interest and so forth.

In still other embodiments, the sensor array can employ multiple ionic species sensors 26 having differing films 66, which would therefore alter the electrical information supplied to the controller 34 by each ionic species sensor 26. For example, differing ionic species sensors 26 can be employed to reduce interference caused by the presence of contaminants other than the particular non-desired contaminant to be sensed within the liquid sample for the purpose of increasing the accuracy of a measurement of the particular non-desired contaminant within the liquid sample.

To be more specific, the first ionic species sensor 26A can be employed to provide electrical information to the controller 34 based on a first contaminant, which is the non-desired contaminant of interest to be measured. However, if a second and third contaminant are known to obscure the accuracy of the first contaminant concentration within the liquid sample, the sensor array can be configured with the second ionic species sensor 26B configured to sense the second contaminant, and a third ionic species sensor 26C (not shown) configured to sense the third contaminant. In such implementations, the electrical information supplied by the three ionic species sensors 26A, 26B and 26C can be utilized and analyzed by the controller 34, via execution of the respective CCD algorithm. If the controller 34 determines that the second ionic species sensor 26B did not detect the second contaminant, and the third ionic species sensor 26C did not detect the third contaminant, the information received from the first ionic species sensor 26A is determined to be accurate and not obscured by the presence of the second or third contaminant. However, if the presence of either the second contaminant or the third contaminant is determined, the controller 34 can account for the concentration of these contaminants to determine the accurate concentration of the first contaminant within the liquid sample.

In various embodiments, to determine the concentration of the non-desired contaminant in the liquid sample in accordance with the selected CCD algorithm, the CCD algorithm can implement multivariate analysis. More particularly, the selected CCD algorithm can employ multivariate analysis tools to determine the concentration of the non-desired contaminant in the liquid sample utilizing the electrical information from each of the ionic species sensors 26 in the array. The selected CCE algorithm can employ any suitable multivariate analysis tool, such as canonical correlation analysis, regression analysis, principal components analysis, discriminant function analysis, multidimensional scaling, linear discriminant analysis, logistic regression, and/or neural network analysis.

When describing elements or features of the present disclosure or embodiments thereof, the articles "a", "an", "the", and "said" are intended to mean that there are one or more of the elements or features. The terms "comprising", "including", and "having" are intended to be inclusive and mean that there may be additional elements or features beyond those specifically described.

The description herein is merely exemplary in nature and, thus, variations that do not depart from the gist of that which is described are intended to be within the scope of the teachings. Such variations are not to be regarded as a departure from the spirit and scope of the teachings.

It is further to be understood that the processes or steps described herein are not to be construed as necessarily requiring their performance in the particular order discussed or illustrated. It is also to be understood that additional or alternative processes or steps may be employed.

For completeness, various aspects of the invention are now set out in the following numbered clauses:
1. A method for detecting contaminants in a liquid, said method comprising:
   filling at least a portion of a sample container interior chamber with a liquid sample;
   submerging a sensor probe of a hand-held portable sensing device in a liquid sample;
   sensing an electrical conductivity of the liquid sample utilizing at least one conductivity sensor attached to the submerged sensor probe of the hand-held portable sensing device and electrically connected to a controller of the hand-held portable sensing device;
   automatically selecting, based on the sensed conductivity, a particular one of a plurality of contaminant concentration detection (CCD) algorithms stored on the memory device of the hand-held portable sensing device;
   setting a sensitivity of at least one ionic species sensor to a sensitivity level particular to the selected CCD algorithm, each CCD algorithm configured to implement a particular sensitivity setting associated with the respective CCD algorithm, the at least one ionic species sensor attached to the submerged sensor probe of the hand-held portable sensing device and electrically connected to the controller of the hand-held portable sensing device;
   sensing non-desired contaminants in the liquid sample utilizing the at least one ionic species sensor set to the particular sensitivity level; and
   determining a concentration of the non-desired contaminant in the liquid sample in accordance with the selected CCD algorithm.
2. The method of clause 1, wherein determining a concentration of the non-desired contaminant in the liquid sample in accordance with the selected CCD algorithm comprises:
   sensing a temperature of the liquid sample utilizing a temperature sensor attached to the sensor probe of the hand-held portable sensing device and electrically connected to the controller; and
   determining the concentration of the non-desirable contaminant utilizing the sensed temperature of the liquid sample.
3. The method of clause 2 further comprising displaying at least one of the sensed electrical conductivity of the liquid sample, the temperature of the liquid sample and the concentration of the non-desired contaminant in the liquid sample utilizing a display included in a head of the hand-held portable sensing device and electrically connected to the controller.
4. The method of clause 1, wherein determining a concentration of the non-desired contaminant in the liquid sample in accordance with the selected CCD algorithm comprises:
   sensing a temperature of the liquid sample utilizing a temperature sensor attached to the sensor probe of the hand-held portable sensing device and electrically connected to the controller;
   maintaining the liquid sample at a substantially constant temperature over a period of time utilizing the temperature sensor and a thermal electric heating and cooling device attached to the sensor probe of the hand-held portable sensing device and electrically connected to the controller;
   acquiring multiple non-desired contaminants readings over the period utilizing the at least one ionic species sensor; and
   determining the concentration of the non-desirable contaminant utilizing multiple contaminant readings.
5. The method of clause 1, wherein determining a concentration of the non-desired contaminant in the liquid sample in accordance with the selected CCD algorithm comprises:
   sensing a temperature of the liquid sample utilizing a temperature sensor attached to the sensor probe of the hand-held portable sensing device and electrically connected to the controller;
   cycling the temperature of the liquid sample between two or more temperatures over a period of time utilizing the temperature sensor and a thermal electric heating and cooling device attached to the sensor probe of the hand-held portable sensing device and electrically connected to the controller;
   acquiring multiple non-desired contaminant readings over the period utilizing the at least one ionic species sensor; and
   determining the concentration of the non-desirable contaminant utilizing multiple contaminant readings.
6. The method of clause 1, wherein determining a concentration of the non-desired contaminant in the liquid sample in accordance with the selected CCD algorithm comprises:
   sensing an amount of organic carbon in the liquid sample utilizing an organic carbon sensor attached to the sensor probe of the hand-held portable sensing device and electrically connected to the controller; and
   determining the concentration of the non-desirable contaminant utilizing the amount of sensed organic carbon in the liquid sample.
7. The method of clause 1 further comprising utilizing a stored power device included in a head of the hand-held portable sensing device to provide electrical power to:
   the controller,
   the at least one conductivity sensor,
   the at least one ionic species sensor, and
   at least one of a temperature sensor attached to the sensor probe of the hand-held portable sensing device and electrically connected to the controller, a display included in a head of the hand-held portable sensing device and electrically connected to the controller, and a thermal electric heating and cooling device attached to the sensor probe of the hand-held portable sensing device electrically connected to the controller.
8. The method of clause 7 further comprising executing a power saving subroutine of the selected CCD algorithm to control consumption of power from the stored power device by the controller, the at least one conductivity sensor, the at least one ionic species sensor, the temperature sensor, the thermal electric heating and cooling device, and the display during use of the hand-held portable sensing device.
9. The method of clause 1, wherein the at least one ionic species sensor comprising a plurality of ionic species sensors forming an array of ionic species sensors attached to the sensor probe of the hand-held portable sensing device and electrically connected to the controller, each ionic species sensor independently sensing respective independent values of the non-desired contaminant in the liquid sample, and
   wherein determining a concentration of the non-desired contaminant in the liquid sample in accordance with the selected CCD algorithm comprises implementing multivariate analysis to determine the concentration of the non-desirable contaminant.
10. The method of clause 1, wherein the sample container comprises an air-tight container having the interior chamber under a vacuum, and filling at least a portion of the sample container interior chamber with a liquid sample comprises:
   coupling the hand-held portable sensing device in an air-tight fashion to a first end of the air-tight container having the at least one conductivity sensor and the at least one ionic species sensor positioned within the interior chamber of the container;
   exposing a tubular stem at an opposing second end of the air-tight container to a liquid to be sampled; and
   breaking an air-tight seal within the tubular stem such that the liquid sample is drawn into the interior chamber of the container, thereby preventing exposure of the liquid sample to ambient impurities and preserving the integrity of the liquid sample.
11. The method of clause 1, wherein the sample container comprises an air-tight piston and cylinder, and filling at least a portion of the sample container interior chamber with a liquid sample comprises:
   coupling the hand-held portable sensing device including the at least one conductivity sensor and the at least one ionic species sensor to the base of the piston placed within the cylinder;
   exposing a tubular stem at an opposing end of the air-tight cylinder to the liquid to be sampled;
   withdrawing the piston to a desired position to create a vacuum in the cylinder such that the liquid sample is drawn through the tubular stem into the cylinder such that the sensors come in contact with the liquid sample, thereby preventing exposure of the liquid sample to ambient impurities, preserving the integrity of the liquid sample, and ensuring a precise volume is retained.
12. The method of clause 1, wherein the container comprises a microwell having the at least one conductivity sensor and at least one ionic species sensor arranged on a bottom of the microwell, and wherein filling at least a portion of a sample container interior chamber with a liquid sample is performed substantially simultaneously with submerging a sensor probe of a hand-held portable sensing device in a liquid sample.
13. The method of clause 1, wherein determining a concentration of the non-desired contaminant in the liquid sample in accordance with the selected CCD algorithm comprises:
   sensing a temperature of the liquid sample utilizing a temperature sensor attached to the sensor probe of the hand-held portable sensing device and electrically connected to the controller;
   sensing an organic carbon concentration of the liquid sample utilizing an organic carbon sensor attached to the sensor probe of the hand-held portable sensing device and electrically connected to the controller; and
   determining the concentration of the non-desirable contaminant taking into account the information from the sensed temperature and organic carbon level of the liquid sample.
14. The method of clause 1, further comprising:
   utilizing the determined concentrations of the non-desired contaminants during execution of the selected CCD algorithm to estimate the conductivity of the liquid sample;
   comparing the estimated conductivity to the sensed conductivity; and
   generating an error alert, indicating that a possible error has occurred, when a differential between the estimated conductivity and the sensed conductivity is greater than a specific value.
15. A hand-held, portable system for detection of contaminants in a liquid, said system comprising:
   a sample container for retaining a liquid sample; and
   a hand-held portable sensing device at least partially submersible into the liquid sample and operable for determining a concentration of a non-desired contaminant in the liquid sample, the hand-held portable sensing comprising:
      at least one conductivity sensor for sensing an electrical conductivity of the liquid sample,
      at least one ionic species sensor for sensing the non-desired contaminant in the liquid sample, and
      a controller electrically connected to the at least one conductivity sensor and the at least one ionic species sensor, the controller including:
         a processor, and
         a computer readable memory device having stored thereon a contaminant concentration detection (CCD) algorithm selection routine executable by the processor to determine an electrical conductivity value of the liquid sample and, based on the determined conductivity value, instruct the processor to execute a particular one of a plurality of CCD algorithms stored on the memory device, each respective CCD algorithm configured to determine a concentration of the non-desired contaminant in the liquid sample using a respective different sensitivity setting for the at least one ionic species sensor.
16. The system of clause 15, wherein the hand-held portable sensing device further comprises a temperature sensor electrically connected to the controller for sensing a temperature of the liquid sample and each of the CCD algorithms are configured to determine the concentration of the non-desirable contaminant utilizing a sensed temperature of the liquid sample.
17. The system of clause 16, wherein the hand-held portable sensing device further comprises a thermal electric heating and cooling device electrically connected to the controller for heating and cooling the liquid sample and each of the CCD algorithms are configured to utilize the thermal electric heating and cooling device to maintain the liquid sample at a substantially constant temperature over a period of time and take multiple contaminant readings over the period to determine the concentration of the non-desirable contaminant utilizing the multiple contaminant readings.
18. The system of clause 16, wherein the hand-held portable sensing device further comprises a thermal electric heating and cooling device electrically connected to the controller for heating and cooling the liquid sample and each of the CCD algorithms are configured to utilize the thermal electric heating and cooling device to cycle the temperature of the liquid sample between two or more temperatures over a period of time and take multiple contaminant readings at the different temperatures over the period to determine the concentration of the non-desirable contaminant utilizing the multiple contaminant readings.
19. The system of clause 16, wherein the hand-held portable sensing device further comprises a display electrically connected to the controller for displaying at least one of the sensed electrical conductivity of the liquid sample, the temperature of the liquid sample and the concentration of the non-desired contaminant in the liquid sample.
20. The system of clause 15, wherein the hand-held portable sensing device further comprises an organic carbon sensor electrically connected to the controller for sensing an amount of organic carbon in the liquid sample and each of the CCD algorithms are configured to determine the concentration of the non-desirable contaminant utilizing a sensed amount of organic carbon in the liquid sample.
21. The system of clause 15, wherein the hand-held portable sensing device further comprises:
   at least one of:
      a display electrically connected to the controller for displaying at least one the sensed electrical conductivity of the liquid sample and the concentration of the non-desired contaminant in the liquid sample, and
      a thermal electric heating and cooling device electrically connected to the controller for heating and cooling the liquid sample, and
      a stored power device for providing electrical power to the controller, the display, the thermal electric heating device, the conductivity sensor and the ionic species sensor,
      each of the CCD algorithms including a power saving subroutine for controlling power consumption by the controller, the sensors, thermal electric heating and cooling device and display during use of the hand-held portable sensing device.
22. The system of clause 15 comprising a ionic species sensor array including a plurality of ionic species sensors electrically connected to the controller, each ionic species sensor independently sensing a respective independent value of the non-desired contaminant in the liquid sample, and at least one of the CCD algorithms is configured to implement multivariate analysis to determine the concentration of the non-desirable contaminant.
23. The system of clause 15, wherein the sample container comprises an air-tight container coupleable at a first end to the hand-held portable sensing device in an air-tight fashion having the at least one conductivity sensor and the at least one ionic species sensor positioned within an interior chamber of the container, the interior chamber being under a vacuum, the container comprising a tubular stem and air-tight seal at an opposing second end such that when the stem is exposed to a liquid and the air-tight seal is broken, the liquid sample will be drawn into the interior chamber of the container to prevent exposure of the liquid sample to ambient impurities and preserve the integrity of the liquid sample.
24. The system of clause 15, wherein the sample container comprises an air-tight piston and cylinder, with the hand-held portable sensing device having the at least one conductivity sensor and the at least one ionic species sensor embedded in a base of the piston, the cylinder comprising a tubular stem for inserting into a volume of a liquid to be sampled, such that when the piston is withdrawn to a desired position a vacuum is created in the cylinder drawing the liquid through the tubular stem into the cylinder to provide the liquid sample, thereby preventing exposure of the liquid sample to ambient impurities, preserving the integrity of the liquid sample, and ensuring a precise volume is retained.
25. The system of clause 15, wherein the container comprises a microwell having the at least one conductivity sensor and at least one ionic species sensor arranged on a bottom of the microwell, and wherein the sensor probe is submerged into the liquid sample substantially simultaneously with filling at least a portion of a sample container interior chamber with a liquid sample.
26. The system of clause 15, wherein the selected CCD algorithm is further configured to:
   sense a temperature of the liquid sample utilizing a temperature sensor attached to the sensor probe of the hand-held portable sensing device and electrically connected to the controller;
   sense an organic carbon concentration of the liquid sample utilizing an organic carbon sensor attached to the sensor probe of the hand-held portable sensing device and electrically connected to the controller; and
   determine the concentration of the non-desirable contaminant taking into account the information from the sensed temperature and organic carbon level of the liquid sample.
27. The system of clause 15, wherein the selected CCD algorithm is further configured to:
   utilize the determined concentrations of the non-desired contaminants during to estimate the conductivity of the liquid sample;
   compare the estimated conductivity to the sensed conductivity; and
   generate an error alert, indicating that a possible error has occurred, when a differential between the estimated conductivity and the sensed conductivity is greater than a specific value.
28. A method for detecting contaminants in a liquid, said method comprising:
   submerging a sensor probe of a hand-held portable sensing device in a liquid sample retained within a sample container;
   sensing an electrical conductivity of the liquid sample utilizing a conductivity sensor attached to the sensor probe of the hand-held portable sensing device and electrically connected to a controller of the hand-held portable sensing device;
   automatically selecting, based on the sensed conductivity, a particular one of a plurality of contaminant concentration detection (CCD) algorithms stored on the memory device of the hand-held portable sensing device;
   setting a sensitivity of each of a plurality of sensors in an ionic species sensor array to a sensitivity level particular to the selected CCD algorithm, each CCD algorithm configured to implement a particular sensitivity setting associated with the respective CCD algorithm, the at least one ionic species sensor attached to the sensor probe of the hand-held portable sensing device and electrically connected to the controller of the hand-held portable sensing device;
   sensing a temperature of the liquid sample utilizing a temperature sensor attached to the sensor probe of the hand-held portable sensing device and electrically connected to the controller;
   sensing non-desired contaminants in the liquid sample utilizing ionic species sensor array having each sensor set to the particular sensitivity level; and
   determining a concentration of the non-desired contaminant in the liquid sample in accordance with the selected CCD algorithm utilizing non-desired contaminants readings from the ionic species sensor array and temperature readings from the temperature sensor.
29. The method of clause 28 further comprising displaying at least one of the sensed electrical conductivity of the liquid sample, the temperature of the liquid sample and the concentration of the non-desired contaminant in the liquid sample utilizing a display included in a head of the hand-held portable sensing device and electrically connected to the controller.
30. The method of clause 28, wherein determining a concentration of the non-desired contaminant in the liquid sample in accordance with the selected CCD algorithm comprises:
   sensing a temperature of the liquid sample utilizing a temperature sensor attached to the sensor probe of the hand-held portable sensing device and electrically connected to the controller;
   maintaining the liquid sample at a substantially constant temperature over a period of time utilizing the temperature sensor and a thermal electric heating and cooling device attached to the sensor probe of the hand-held portable sensing device and electrically connected to the controller;
   acquiring multiple non-desired contaminant readings over the period utilizing the ionic species sensor array; and
   determining the concentration of the non-desirable contaminant utilizing multiple contaminant readings.
31. The method of clause 28, wherein determining a concentration of the non-desired contaminant in the liquid sample in accordance with the selected CCD algorithm comprises:
   sensing a temperature of the liquid sample utilizing a temperature sensor attached to the sensor probe of the hand-held portable sensing device and electrically connected to the controller;
   cycling the temperature of the liquid sample between two or more temperatures over a period of time utilizing the temperature sensor and a thermal electric heating and
   cooling device attached to the sensor probe of the hand-held portable sensing device and electrically connected to the controller;
   acquiring multiple non-desired contaminants readings over the period utilizing the ionic species sensor array; and
   determining the concentration of the non-desirable contaminant utilizing multiple contaminant readings.
32. The method of clause 28, wherein determining a concentration of the non-desired contaminant in the liquid sample in accordance with the selected CCD algorithm comprises:
   sensing an amount of organic carbon in the liquid sample utilizing an organic carbon sensor attached to the sensor probe of the hand-held portable sensing device and electrically connected to the controller; and
   determining the concentration of the non-desirable contaminant utilizing the amount of sensed organic carbon in the liquid sample.
33. The method of clause 28 further comprising utilizing a stored power device included in a head of the hand-held portable sensing device to provide electrical power to:
   the controller, the conductivity sensor, the ionic species sensor array, and
   at least one of a temperature sensor attached to the sensor probe of the hand-held portable sensing device and electrically connected to the controller, a display included in a head of the hand-held portable sensing device and electrically connected to the controller, and a thermal electric heating and cooling device attached to the sensor probe of the hand-held portable sensing device electrically connected to the controller.
34. The method of clause 33 further comprising executing a power saving subroutine of the selected CCD algorithm to control consumption of power from the stored power device by the controller, the at least one conductivity sensor, the at least one ionic species sensor, the temperature sensor, the thermal electric heating and cooling device, and the display during use of the hand-held portable sensing device.

## Claims

1. A method for detecting contaminants in a liquid, said method comprising:
filling at least a portion of a sample container interior chamber with a liquid sample;
submerging a sensor probe of a hand-held portable sensing device in a liquid sample;
sensing an electrical conductivity of the liquid sample utilizing at least one conductivity sensor attached to the submerged sensor probe of the hand-held portable sensing device and electrically connected to a controller of the hand-held portable sensing device;
automatically selecting, based on the sensed conductivity, a particular one of a plurality of contaminant concentration detection (CCD) algorithms stored on the memory device of the hand-held portable sensing device;
setting a sensitivity of at least one ionic species sensor to a sensitivity level particular to the selected CCD algorithm, each CCD algorithm configured to implement a particular sensitivity setting associated with the respective CCD algorithm, the at least one ionic species sensor attached to the submerged sensor probe of the hand-held portable sensing device and electrically connected to the controller of the hand-held portable sensing device;
sensing non-desired contaminants in the liquid sample utilizing the at least one ionic species sensor set to the particular sensitivity level; and
determining a concentration of the non-desired contaminant in the liquid sample in accordance with the selected CCD algorithm.

2. The method of Claim 1, wherein determining a concentration of the non-desired contaminant in the liquid sample in accordance with the selected CCD algorithm comprises:
sensing a temperature of the liquid sample utilizing a temperature sensor attached to the sensor probe of the hand-held portable sensing device and electrically connected to the controller; and
determining the concentration of the non-desirable contaminant utilizing the sensed temperature of the liquid sample.

3. The method of Claim 2 further comprising displaying at least one of the sensed electrical conductivity of the liquid sample, the temperature of the liquid sample and the concentration of the non-desired contaminant in the liquid sample utilizing a display included in a head of the hand-held portable sensing device and electrically connected to the controller.

4. The method of Claim 1, wherein determining a concentration of the non-desired contaminant in the liquid sample in accordance with the selected CCD algorithm comprises:
sensing a temperature of the liquid sample utilizing a temperature sensor attached to the sensor probe of the hand-held portable sensing device and electrically connected to the controller;
maintaining the liquid sample at a substantially constant temperature over a period of time utilizing the temperature sensor and a thermal electric heating and cooling device attached to the sensor probe of the hand-held portable sensing device and electrically connected to the controller;
acquiring multiple non-desired contaminants readings over the period utilizing the at least one ionic species sensor; and
determining the concentration of the non-desirable contaminant utilizing multiple contaminant readings.

5. The method of Claim 1, wherein determining a concentration of the non-desired contaminant in the liquid sample in accordance with the selected CCD algorithm comprises:
sensing a temperature of the liquid sample utilizing a temperature sensor attached to the sensor probe of the hand-held portable sensing device and electrically connected to the controller;
cycling the temperature of the liquid sample between two or more temperatures over a period of time utilizing the temperature sensor and a thermal electric heating and cooling device attached to the sensor probe of the hand-held portable sensing device and electrically connected to the controller;
acquiring multiple non-desired contaminant readings over the period utilizing the at least one ionic species sensor; and
determining the concentration of the non-desirable contaminant utilizing multiple contaminant readings.

6. The method of Claim 1, wherein determining a concentration of the non-desired contaminant in the liquid sample in accordance with the selected CCD algorithm comprises:
sensing an amount of organic carbon in the liquid sample utilizing an organic carbon sensor attached to the sensor probe of the hand-held portable sensing device and electrically connected to the controller; and
determining the concentration of the non-desirable contaminant utilizing the amount of sensed organic carbon in the liquid sample.

7. The method of any of the preceding claims, further comprising utilizing a stored power device included in a head of the hand-held portable sensing device to provide electrical power to:
the controller,
the at least one conductivity sensor,
the at least one ionic species sensor, and
at least one of a temperature sensor attached to the sensor probe of the hand-held portable sensing device and electrically connected to the controller, a display included in a head of the hand-held portable sensing device and electrically connected to the controller, and a thermal electric heating and cooling device attached to the sensor probe of the hand-held portable sensing device electrically connected to the controller.

8. The method of Claim 7 further comprising executing a power saving subroutine of the selected CCD algorithm to control consumption of power from the stored power device by the controller, the at least one conductivity sensor, the at least one ionic species sensor, the temperature sensor, the thermal electric heating and cooling device, and the display during use of the hand-held portable sensing device.

9. The method of any of the preceding claims, wherein the at least one ionic species sensor comprising a plurality of ionic species sensors forming an array of ionic species sensors attached to the sensor probe of the hand-held portable sensing device and electrically connected to the controller, each ionic species sensor independently sensing respective independent values of the non-desired contaminant in the liquid sample, and
wherein determining a concentration of the non-desired contaminant in the liquid sample in accordance with the selected CCD algorithm comprises implementing multivariate analysis to determine the concentration of the non-desirable contaminant.

10. The method of any of the preceding claims, wherein the sample container comprises an air-tight container having the interior chamber under a vacuum, and filling at least a portion of the sample container interior chamber with a liquid sample comprises:
coupling the hand-held portable sensing device in an air-tight fashion to a first end of the air-tight container having the at least one conductivity sensor and the at least one ionic species sensor positioned within the interior chamber of the container;
exposing a tubular stem at an opposing second end of the air-tight container to a liquid to be sampled; and
breaking an air-tight seal within the tubular stem such that the liquid sample is drawn into the interior chamber of the container, thereby preventing exposure of the liquid sample to ambient impurities and preserving the integrity of the liquid sample.

11. The method of any of the preceding claims, wherein the sample container comprises an air-tight piston and cylinder, and filling at least a portion of the sample container interior chamber with a liquid sample comprises:
coupling the hand-held portable sensing device including the at least one conductivity sensor and the at least one ionic species sensor to the base of the piston placed within the cylinder;
exposing a tubular stem at an opposing end of the air-tight cylinder to the liquid to be sampled;
withdrawing the piston to a desired position to create a vacuum in the cylinder such that the liquid sample is drawn through the tubular stem into the cylinder such that the sensors come in contact with the liquid sample, thereby preventing exposure of the liquid sample to ambient impurities, preserving the integrity of the liquid sample, and ensuring a precise volume is retained.

12. The method of any of the preceding claims, wherein the container comprises a microwell having the at least one conductivity sensor and at least one ionic species sensor arranged on a bottom of the microwell, and wherein filling at least a portion of a sample container interior chamber with a liquid sample is performed substantially simultaneously with submerging a sensor probe of a hand-held portable sensing device in a liquid sample.

13. The method of any of the preceding claims, wherein determining a concentration of the non-desired contaminant in the liquid sample in accordance with the selected CCD algorithm comprises:
sensing a temperature of the liquid sample utilizing a temperature sensor attached to the sensor probe of the hand-held portable sensing device and electrically connected to the controller;
sensing an organic carbon concentration of the liquid sample utilizing an organic carbon sensor attached to the sensor probe of the hand-held portable sensing device and electrically connected to the controller; and
determining the concentration of the non-desirable contaminant taking into account the information from the sensed temperature and organic carbon level of the liquid sample.

14. A hand-held, portable system for detection of contaminants in a liquid, said system comprising:
a sample container for retaining a liquid sample; and
a hand-held portable sensing device at least partially submersible into the liquid sample and operable for determining a concentration of a non-desired contaminant in the liquid sample, the hand-held portable sensing comprising:
at least one conductivity sensor for sensing an electrical conductivity of the liquid sample,
at least one ionic species sensor for sensing the non-desired contaminant in the liquid sample, and
a controller electrically connected to the at least one conductivity sensor and the at least one ionic species sensor, the controller including:
a processor, and
a computer readable memory device having stored thereon a contaminant concentration detection (CCD) algorithm selection routine executable by the processor to determine an electrical conductivity value of the liquid sample and, based on the determined conductivity value, instruct the processor to execute a particular one of a plurality of CCD algorithms stored on the memory device, each respective CCD algorithm configured to determine a concentration of the non-desired contaminant in the liquid sample using a respective different sensitivity setting for the at least one ionic species sensor.

15. A method for detecting contaminants in a liquid, said method comprising:
submerging a sensor probe of a hand-held portable sensing device in a liquid sample retained within a sample container;
sensing an electrical conductivity of the liquid sample utilizing a conductivity sensor attached to the sensor probe of the hand-held portable sensing device and electrically connected to a controller of the hand-held portable sensing device;
automatically selecting, based on the sensed conductivity, a particular one of a plurality of contaminant concentration detection (CCD) algorithms stored on the memory device of the hand-held portable sensing device;
setting a sensitivity of each of a plurality of sensors in an ionic species sensor array to a sensitivity level particular to the selected CCD algorithm, each CCD algorithm configured to implement a particular sensitivity setting associated with the respective CCD algorithm, the at least one ionic species sensor attached to the sensor probe of the hand-held portable sensing device and electrically connected to the controller of the hand-held portable sensing device;
sensing a temperature of the liquid sample utilizing a temperature sensor attached to the sensor probe of the hand-held portable sensing device and electrically connected to the controller;
sensing non-desired contaminants in the liquid sample utilizing ionic species sensor array having each sensor set to the particular sensitivity level; and
determining a concentration of the non-desired contaminant in the liquid sample in accordance with the selected CCD algorithm utilizing non-desired contaminants readings from the ionic species sensor array and temperature readings from the temperature sensor.
